# EUROPEAN PATENT APPLICATION

(11) **EP 2 319 915 A1**
(43) Date of publication of application: **11.05.2011**
(21) Application number: 09175071.1
(22) Date of filing: 04.11.2009
(51) Int. Cl.: C12N 5/071

(54) **A novel conditionally immortalized human proximal tubule cell line expressing functional influx and efflux transporters.**

(71) Applicant: Stichting Katholieke Universiteit, 6500 HB Nijmegen (NL)
(72) Inventor: Levtchenko, Elena Nikolaevna, 6525 EZ, NIJMEGEN (NL); van den Heuvel, Lambertus Petrus Wilhelmus Johann, 6525 EZ, NIJMEGEN (NL); Wilmer, Martijn Jozef Gerardus, 6525 EZ, NIJMEGEN (NL); Russel, Francois Gérard Marie, 6525 EZ, NIJMEGEN (NL)
(74) Representative: Habets, Winand

(57) **Abstract**

The invention relates to the field of biology and medicine. In particular, the invention relates to the field of pharmaceutical research and drug development, and especially to means for physiological, pharmacological and toxicological research. More in particular, the invention provides renal cell lines with proximal tubular characteristics including multiple influx and efflux transporters. These cell lines are termed ciPTEC. Four representative examples have been deposited at the Deutsche Sammlung von Mikroorganismen und Zellkulturen GmbH (DSMZ or German Collection of Microorganisms and Cell Cultures) at Inhoffenstraße 7 B, 38124 Braunschweig, Germany under accession number DSM ACC3019, DSM ACC3020, DSM ACC3021 and DSM ACC3022.

## Description

### Field of the invention

The invention relates to the field of biology and medicine. In particular, the invention relates to the field of pharmaceutical research and drug development, and especially to means for physiological and pharmacological research. More in particular, the invention provides a renal cell line with proximal tubular characteristics including multiple influx and efflux transporters.

### Background of the invention

In the kidney, the proximal tubular epithelium is responsible for reabsorption of filtered solutes and excretion of waste products and xenobiotics. Numerous solutes, such as phosphate, urate, glucose, and amino acids, are filtered in the glomerulus and reabsorbed in the proximal tubules by carrier-mediated transport, driven by an electrochemical gradient [13]. Other compounds of the glomerular filtrate, such as albumin and low molecular weight proteins, are reabsorbed by receptor-mediated endocytosis [11]. The excretion of metabolic waste products or drugs and their biotransformation products is facilitated by multiple organic ion transporters mediating uptake (influx) from blood at the basolateral membrane and efflux across the apical membrane of proximal tubular epithelial cells (PTEC) [21].

Specific transporters belonging to solute carrier (SLC) and ATP-binding cassette (ABC) family are expressed in the proximal tubular cells of the kidney and mediate renal excretion of diverse various endogenous and exogenous compounds from the body. ATP-binding cassette (ABC) transporters are efflux transporters because they use energy derived from ATP hydrolysis to mediate the active export of drugs. Many of the SLC family members facilitate the cellular influx of substrates, either by facilitated diffusion or active transport coupled to the symport or antiport of inorganic or small organic ions.

These transporters are of considerable pharmacological and toxicological interest, because their substrates encompass among others uremic toxins and numerous drugs including antibiotic, antidiabetic, anti-inflammatory, antiviral, chemotherapeutic, cardiovascular, central nervous system, and immunosuppressant drugs.

The function and regulation of these transport systems is subject of physiological, pharmacological, and toxicological research and requires a suitable in vitro cell model.

The development of experimental kidney model systems, particularly those derived from humans, expressing functional drug transporters is of paramount importance for the identification of substrates and inhibitors of renal drug excretion and predicting potential renal drug-drug interactions.

A major drawback for the production of a renal cell line that expresses solute carrier (SLC) and ATP-binding cassette (ABC) family members, is that the expression of many of said family members is gradually lost upon immortalization of said cell line. Therefore, there exists a clear need for the generation of a human renal cell line that expresses solute carrier (SLC) and ATP-binding cassette (ABC) family members.

In the last two decades, a variety of human and animal renal tubular cell lines have been used for this purpose, recently reviewed by Bens et al [1].

Current cell lines that are being used for pharmacological studies include NRK-52E cells, a normal rat kidney cell line composed of proximal tubular cells, which is however not useful because of large species difference between rat and human in renal drug transport.

Also frequently used are Caco-2 cells, a human colon carcinoma cell line which is not useful for renal drug transport because it expresses intestinal drug transporters.

Currently available human PTEC have variable characteristics or express only few transporters, such as the commercially available HK-2 cell line, obtained from renal cortex and transfected with recombinant HPV16 E6/E7 genes [22,18]. HK-2 cells, however, lack the functional expression of the most important drug transport systems.

Primary PTEC, isolated from either human or animal kidney material, can only yield a limited amount of material, as proliferation stops at a few passages and cells dedifferentiate [26,29,4].

The development of a human cell model with proximal tubular characteristics, including multiple influx and efflux transporters, would be useful in the research of renal solute reabsorption and drug excretion and consequently in the in vitro identification of substrates and inhibitors of renal drug transport and the prediction of potential drug-drug interactions at the level of renal clearance. Hence, it may allow the development of pharmaceutical preparations that may overcome renal failure.

### Summary of the invention

The present invention addresses this problem in that it provides conditionally immortalized human proximal tubule cell lines expressing MRP4/ABCC4, in concert with expression and activity of OCT2 and Pgp.

More in detail, the cell lines according to the invention have the following characteristics:
- Cobblestone morphology
- Expression of zona-occludens1 (ZO1), epitheliale tight junction marker
- Resistance of monolayer on permeable filters
- Transepithelial electrochemical resistance (TEER) of monolayer
- Expression of aminopeptidase N, enzyme on proximal brush border
- Alkaline phosphatase activity, enzyme on proximal brush border
- Expression of specific proximal tubular proteins:
   o aquaporin1 (AQP1)
   o organic cation transporter 2 (OCT2/SLC22A2)
   o dipeptidyl-peptidase IV (dppIV)
   o multidrug resistance protein 4 (MRP4/ABCC4)
   o P-glycoprotein (Pgp/MDR1/ABCB1)
- Albumine endocytosis, specifically inhibited by receptor associated protein (RAP)
- Sodium-dependent phosphate uptake
- Functional basolateral transport of substrate ASP via OCT2/SLC22A2
- Functional efflux transport of calceïne via P-glycoprotein/MDR1/ABCB1
- Proliferation of more than 40 passage numbers, while maintaining morphology and expression of AQP1, Pgp/MDR1/ABCB1 and OCT2/SLC22A2 and sodium-dependent phosphate uptake and Pgp-activity.

These cell lines are termed conditionally immortalized PTEC (ciPTEC) and 4 examples of such a cell line have been deposited at the Deutsche Sammlung von Mikroorganismen und Zellkulturen GmbH (DSMZ or German Collection of Microorganisms and Cell Cultures) at Inhoffenstraße 7 B, 38124 Braunschweig, Germany under accession numbers DSM ACC3019, DSM ACC3020, DSM ACC3021and DSM ACC3022.

The invention therefore also relates to a cell line as deposited with the DSMZ under accession number DSM ACC3019, DSM ACC3020, DSM ACC3021or DSM ACC3022

The invention also relates to the use of said cell lines for the functional analysis of renal transporters.

### Detailed description of the invention

Using a non-invasive technique of obtaining renal material from urine, we have developed conditionally immortalized human PTEC (ciPTEC) cell lines from a healthy volunteer. These cell lines may be maintained for at least 45 passages and presents proximal tubular characteristics when cultured at a non-permissive temperature of 37°C for 10 days. The activities of the apical (brush border membrane) ATP-binding cassette (ABC) transporter P-glycoprotein (Pgp/MDR1/ABCB1) and the basolateral membrane solute carrier (SLC) organic cation transporter 2 (OCT2, SLC22A2) as well as the apical uptake of phosphate (mediated by SLC34A1 and SLC34A3) and of albumin are demonstrated. In addition, the presence of the apical proximal tubular-specific ATP-binding cassette (ABC) transporter multidrug resistance protein 4 (MRP4/ABCC4) was confirmed.

Approximately 10% of the 38 collected mid stream urine sediments from 31 healthy volunteers contained viable cells with the ability to proliferate into single cell colonies. Primary cultures showed heterogeneous morphology. Cell cultures infected with SV40T and hTERT were found to be antibiotic-resistant to hygromycin B and geneticin (G418), indicating successful immortalization.

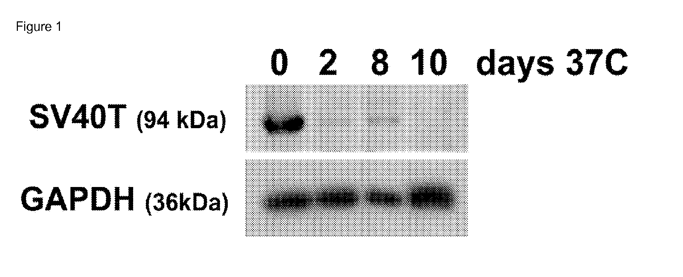

Antibiotic resistance was maintained in ciPTEC cells for at least 40 passages, indicating that expression of SV40T and hTERT remained over time during proliferation at 33°C. Proliferation was maintained at 33°C and cells transferred to 37°C at 70% confluency grew into confluent monolayers within 10 days, while SV40T antigen expression gradually decreased (figure 1).

Subcloning of immortalized cells resulted in a cell culture with homogenous cobblestone morphology. Electron microscopy (EM) analysis showed moderate formation of microvilli and endocytic vesicles. Proliferation of clones could be maintained for at least 45 passages. Routinely, cell morphology was monitored by phase-contrast microscopy at each passage, which showed no marked difference up to passage 40.

To investigate the viability of the cells during maturation at 37°C for 10 days, a resazurin assay was performed [31,16] showing intact viability during the maturation stage (data not shown). Successfully immortalized cell lines obtained from two donors were subcloned, resulting in 15 and 9 clones respectively. These clones were characterized for expression of aminopeptidase N, Pgp/MDR1/ABCB1, aquaporin 1 (AQP1) and dpp-IV expression.

Based on these results, one clone from one donor (female, 12 years) expressing the markers mentioned above, was designated ciPTEC-14.4 (DSM ACC3019) and selected for further investigation.

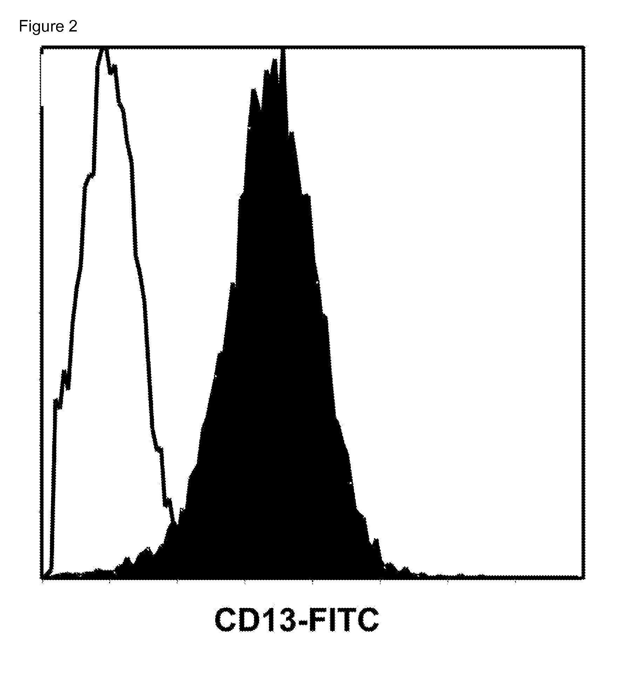

A characterization study was performed after subcloning the cell line to confirm its proximal tubular origin [7]. At 37°C, monolayers of the subclones expressed zona occludens 1 (ZO-1) protein, indicating the epithelial origin of cells with development of tight junctions. Formation of tight monolayers was further supported by the inulin-FITC diffusion experiments. Cells grown on permeable filter supports assembled to a monolayer, with 13.2% (+/-1.1) diffusion of inulin-FITC after 2 hr incubation at 37°C. Proximal tubular specific brush border membrane enzyme aminopeptidase N (CD13) was detected using flow cytometry (figure 2) and confirmed by immuno-fluorescence microscopy

The activity of brush border membrane enzyme alkaline phosphatase was investigated and compared to activity in HK-2 cell lines (ATCC; Manassas VA, USA). In ciPTEC-14.4 the activity of alkaline phosphatase was 0.96 +/- 0.21 and in HK-2 0.59 +/- 0.03 mUnits SAP/mg protein. Endothelial marker CD31 did not bind to ciPTEC.

CiPTEC-14.4 (DSM ACC3019) could be clearly distinguished from human podocyte cell line [23] due to differences in morphology, the presence of CD13 antigen and alkaline phosphatase activity [30].

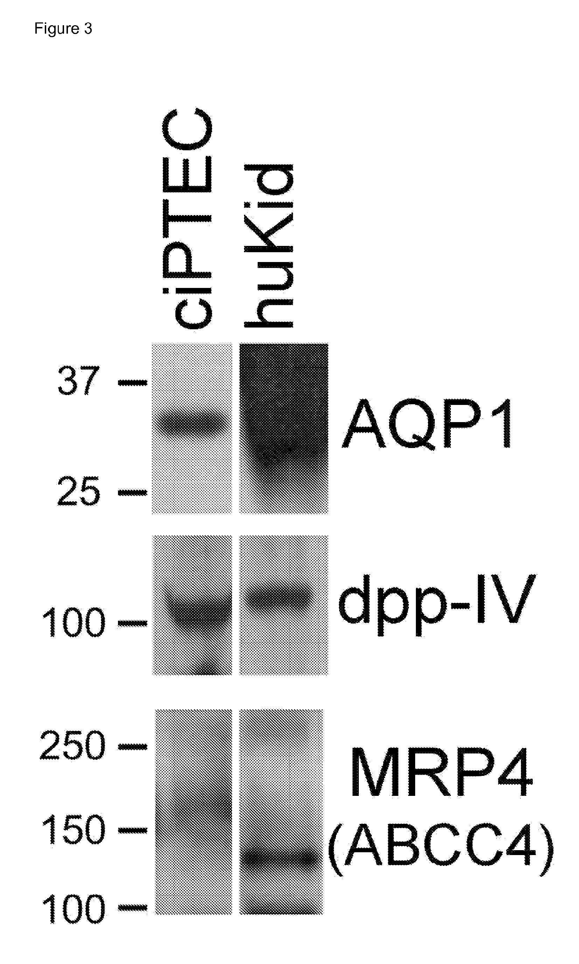

The presence of proximal tubular specific transporters and enzymes AQP1, dpp-IV and MRP4/ABCC4 was demonstrated in cells cultured for 10 days at 37°C (figure 3). Cells originating from distal tubules or collecting ducts were excluded by positive expression of AQP1 in ciPTEC-14.4 [15]. The variations in molecular size between ciPTEC-14.4 and human kidney MRP4 was likely to be due to a difference in glycosylation of this ABC-transporter [8].

The reabsorption of albumin was analyzed using FITC labelled bovine serum albumin (BSA-FITC) uptake in ciPTEC-14-4 between passage number 20-25.

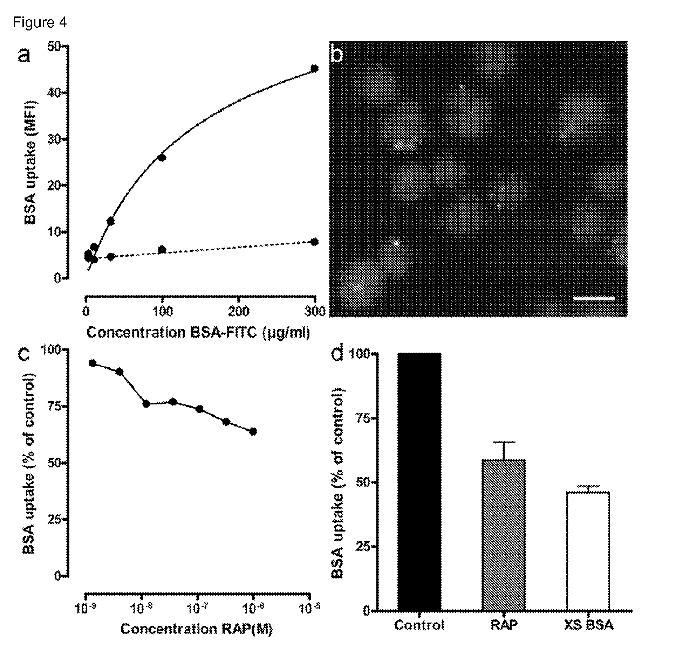

The results show that uptake was concentration- and temperature-dependent, indicating active and specific transport of BSA in ciPTEC-14.4 (figure 4a). Kinetic analysis of BSA-FITC uptake resulted in an apparent Km of 126 µg/ml. To investigate the mechanism of BSA uptake, localization of BSA-FITC was analyzed and uptake was performed in presence of receptor associated protein (RAP), a known inhibitor of albumin endocytosis by binding to multi-ligand receptor megalin [32], or excess unlabelled BSA. The vesicular pattern of BSA-FITC indicates uptake via endocytic vesicles (figure 4b). Additionally, RAP inhibited the uptake of BSA-FITC in a concentration-dependent manner (figure 4c). BSA-FITC uptake was significantly inhibited in the presence of 1 µM RAP (p<0.05) or 200-fold excess unlabelled FITC (p<0.01) by 41 % and 54%, respectively (figure 4d).

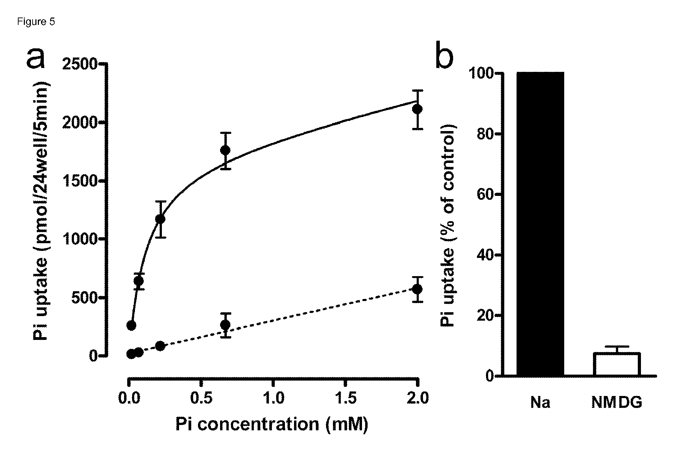

The uptake of phosphate in PTEC is mediated by the sodium-dependent transporters NaPi-IIa (SLC34A1) and NaPi-IIc (SLC34A3) [9]. In the ciPTEC cell lines described herein, the uptake of ³²PO₄ was concentration and sodium-dependent (figure 5a). Maximum phosphate uptake rate (Vmax) was 1717 pmol/24 well/5min and an apparent Km of 0.12 mM was calculated. In the absence of sodium, uptake was significantly decreased by approximately 86% (p<0.001; figure 5b). Phosphate uptake was performed at passage numbers ranging from 30 to 39 with comparable results, suggesting sodium-dependent phosphate uptake remains functional at higher passage numbers.

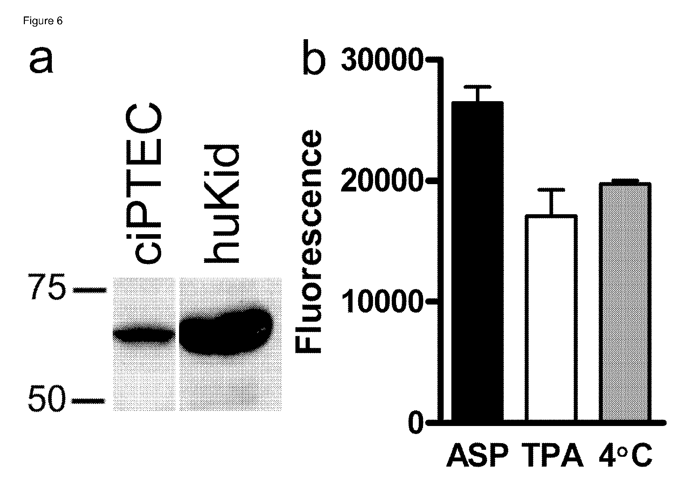

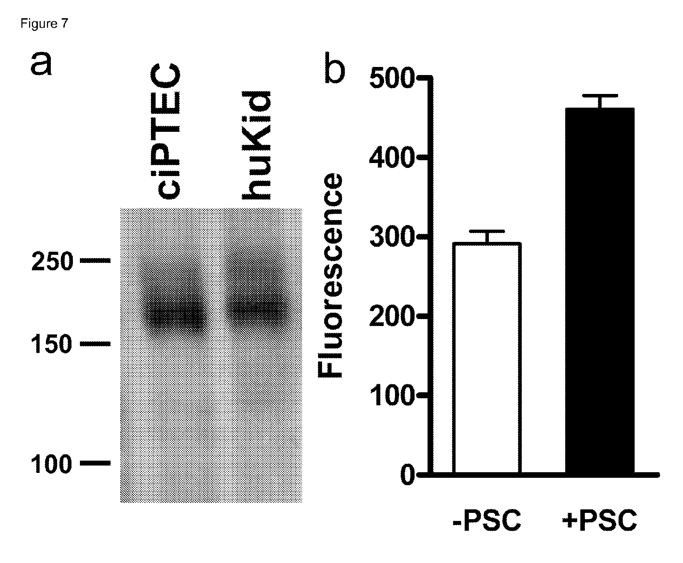

The ability of ciPTEC-14.4 to transport xenobiotics was studied by the expression and activity of the basolateral transporter OCT2/SLC22A2 and the apical efflux ABC transporter Pgp/MDR1/ABCB1. Western blotting using cell homogenates of ciPTEC-14.4 cultured at 37°C clearly showed presence of OCT2 (figure 6a) and Pgp (figure 7a).

Expression of both transporters was confirmed by Western blotting in cell homogenates of passage number 40, indicating tubular characteristics remained over time. Following basolateral exposure, the fluorescent cation 4-(4-(dimethyl-amino)styryl)-N-methylpyridinium iodide (ASP) was internalized by ciPTEC-14.4 cultured on supporting membranes. This transport could be significantly inhibited using OCT2-inhibitor tetrapentylammonium (TPA) (p<0.05) or when uptake was performed at 4°C (p<0.01) by respectively 36 and 30% (figure 6b). Significantly more intracellular fluorescent calcein accumulated when cells were incubated with PSC-833 (ratio 1.6; p<0.001; figure 7b), indicating Pgp-dependent transport activity in ciPTEC.

To determine whether Pgp activity remained over time during proliferation, the calcein assay was performed in cells of up to passage number 39. This resulted in active export of calcein, which could be inhibited by PSC-833 (ratio 2.3), indicating Pgp was functionally expressed at a high passage number.

Three additional cell lines obtained by the process described hereinwere tested for the above parameters and yielded the same results, well within the experimental error margins. These cell lines were termed ciPTEC33.5 (DSM ACC3020), ciPTEC34.8 (DSM ACC3021) and ciPTEC10.3 (DSM ACC3022).

In summary, the ciPTEC cell lines according to the invention have the following characteristics:
- Cobblestone morphology
- Expression of zona-occludens1 (ZO1), epitheliale tight junction marker
- Resistance of monolayer on permeable filters
- Transepithelial electrochemical resistance (TEER) of monolayer
- Expression of aminopeptidase N, enzyme on proximal brush border
- Alkaline phosphatase activity, enzyme on proximal brush border
- Expression of specific proximal tubular proteins:
   o aquaporin1 (AQP1)
   o organic cation transporter 2 (OCT2/SLC22A2)
   o dipeptidyl-peptidase IV (dppIV)
   o multidrug resistance protein 4 (MRP4/ABCC4)
   o P-glycoprotein (Pgp/MDR1/ABCB1)
- Albumine endocytosis, specifically inhibited by receptor associated protein (RAP)
- Sodium-dependent phosphate uptake
- Functional basolateral transport of substrate ASP via OCT2/SLC22A2
- Functional efflux transport of calceïne via P-glycoprotein/MDR1/ABCB1
- Proliferation of more than 40 passage numbers, while maintaining morphology and expression of AQP1, Pgp/MDR1/ABCB1 and OCT2/SLC22A2 and sodium-dependent phosphate uptake and Pgp-activity.

The above results show that we have developed a human conditionally immortalized proximal tubular cell line from urine of a healthy volunteer expressing transporters involved in renal reabsorption and excretion. The immortalization of non-invasively collected cells using SV40T and hTERT vectors enabled us to produce human cells maintaining proximal tubular characteristics, proliferating for at least 45 passages. Expression of SV40T decreased gradually in ciPTEC-14.4 cultured for 10 days at 37°C, minimizing the influence of the transfection on cellular metabolism. Subcloning improved homogeneity of the cell line as is shown by the decrease in morphological variations, probably caused by the exfoliation of various cell types originating from the renal-urinary tract into urine [7].

Culturing PTEC from control urine is usually hampered by low amounts of viable exfoliated cells in urine of healthy volunteers. Interestingly, only some urine portions from a very few subjects contained viable PTEC with variable morphology.

In pharmacology and toxicology, the availability of a cell model of human origin expressing a broad range of functional transporters is of paramount importance. The provision of a cell line according to the invention may facilitate the study of the pathogenesis of inherited proximal tubular disorders, which is often hampered by the limited availability of renal tissue [6,19,5].

More specific, the ciPTEC cell lines as presented herein are the first human cell lines with expression of MRP4/ABCC4, in concert with expression and activity of OCT2 and Pgp. We have confirmed MRP4 expression by quantitative PCR after RNA isolation from ciPTEC. Together with the formation of a tight monolayer, as was observed using the inulin diffusion experiments, these features make ciPTEC cells a valuable tool for identification of substrates and inhibitors of renal drug excretion and the prediction of potential drug-drug interactions in pharmacological research.

Next to functional organic cation excretion, the human ciPTEC cells maintain sodium-dependent phosphate uptake, as well as albumin endocytosis sensitive to inhibition by RAP. The apparent Km calculated for sodium-dependent phosphate transport in ciPTEC-14.4 (0.12 mM) was approximately one-third of the apical phosphate transport value demonstrated in opossum kidney (OK) cells (0.37 mM) [20]. This suggests a higher affinity for phosphate in ciPTEC compared to currently available cell models.

The reabsorption of albumin by ciPTEC in this study is most likely receptor-mediated endocytosis transport, since it is sensitive to RAP inhibition and the intracellular vesicular pattern of BSA-FITC. Inhibition of BSA-FITC in ciPTEC is similar to the inhibition found earlier in OK cells [32], while the apparent Km calculated for ciPTEC-14.4 (126 µg/ml) was approximately 6 times higher than the value reported for OK cells (20µg/ml) [12]. This transport may be facilitated by the multi-ligand receptor megalin, however, we could not identify this receptor by Western blot nor by immunofluorescence techniques (data not shown) [2]. This suggests involvement of alternative albumin reabsorption mechanisms [11].

In conclusion, the present study introduces the first human cell line featuring functional sodium-dependent and endocytosis mediated reabsorption together with functional secretion capacity by Pgp/MDR1/ABCB1 and OCT2/SLC22A2 and combined expression of MRP4/ABCC4. The capacity of ciPTEC to proliferate for extended passages with maintained functional transport, allows for standardized and rapid investigation of renal drug handling and interactions in pharmacological and toxicological research.

A cell line according to the invention may advantageously be used in the functional analysis of renal transporters. It may also be used in a bioassay for testing transport properties of substances through the kidney. In a preferred embodiment, such assays may comprise a solid or semi-solid phase comprising a cell line according the invention wherein said solid or semi-solid phase separates two fluid compartments. For example, said bioassay can be used for assessing epithelial barrier function using, for example a REMS AutoSampler, an Ussing Chamber, or a chopstick electrode/Evometer technique, in the presence or absence of one or more drugs and/or early drug discovery compounds.

In a preferred embodiment, said bioassay can be performed in a medium- or high-throughput method for the identification of substrates of transporters and inhibitors of said transporters, and for predicting potential renal drug-drug interactions. For this, said compartments can be equipped with a fluidic-control system for automatic introduction of compounds, buffers, and gasses into the compartments and sampling from the compartments.

The invention further provides a solid phase comprising a cell line according to the invention. Said solid phase preferably comprises a suitable receptacle such as a tissue culture flask or a multi well plate. Said solid phase can be coated, for example, with collagen or fibronectin

A cell line according to the invention may also be used for specific reabsorption of electrolytes from ultrafiltrate when used in an (bio) artificial kidney. Said cell line may be cultured on highly permeable filters covered with a monolayer of the cells to replace renal function in such a device.

### Legend to the figures

Figure 1: Expression of SV40T in ciPTEC-14.4
   Cell homogenates from ciPTEC-14.4 cultured for various times at 37°C were analyzed for SV40T antigen expression using Western blotting. House-keeping protein GAPDH was used as control.
Figure 2: Proximal tubular epithelial origin of ciPTEC
   Aminopeptidase N was detected using incubation of ciPTEC-14.4 with anti-CD13-FITC and analyzed by flow cytometry (white histogram, negative control; black histogram incubation with CD13-FITC).
Figure 3 Western blotting of ciPTEC-14.4
   Expression of proximal tubule specific proteins aquaporin-1 (AQP1), dipeptidyl peptidase IV (dppIV) and multi resistant protein 4 (MRP4/ABCC4) in cell homogenates of ciPTEC-14.4 were compared with expression in human kidney homogenate (huKid) by Western blotting.
Figure 4 Albumin uptake in ciPTEC-14.4
   Albumin uptake in ciPTEC-14.4 was analyzed using BSA-FITC. (a) Uptake of BSA was concentration- and temperature-dependent (black line, 37°C; dashed line, 4°C); data are expressed as means of duplo experiments. (b) BSA-FITC was located in intracellular vesicles. Bar 10µm (c) Uptake of BSA-FITC (50 µg/ml) was inhibited by RAP in a concentration-dependent manner. Data are expressed as means of duplo experiments. (d) Uptake was significantly inhibited by 1 µM RAP (p<0.05; grey bar) or excess unlabelled BSA (XS BSA, 200-fold; p<0.01; white bar). Data are means of three independent experiments (+/- SE) and expressed as relative uptake compared to normal BSA-FITC uptake.
Figure 5: Sodium-dependent phosphate uptake In ciPTEC
   Uptake of ³²PO₄ (Pi) was analyzed in the presence and absence of sodium in four independent experiments. (a) Uptake of Pi was concentration-dependent and sodium-dependent (in presence of sodium, black line; with NMDG as sodium replacement, dashed line). (b) Uptake of 0.2mM Pi was significantly decreased (p<0.001) in the absence of sodium (NMDG).
Figure 6: OCT2/SLC22A2 activity in ciPTEC
   (a) Presence of OCT2 was shown using Western blotting of ciPTEC-14.4 homogenates. (b) Activity of OCT2 was analyzed by measuring the fluorescence of transported ASP in absence (black bar) or presence (white bar) of OCT2 inhibitor TPA. Additionally, uptake was performed at 4°C (grey bar). Uptake was significantly decreased in ciPTEC-14.4 in presence of TPA (p<0.05) or at 4°C (p<0.01). Data are expressed as mean values +/- SE of three experiments.
Figure 7: P-glycoprotein/MDR1/ABCB1 activity in ciPTEC-14.4
   (a) Presence of Pgp was shown using Western blotting of ciPTEC-14.4 homogenates. (b) Activity of Pgp was analyzed by measuring the fluorescence of accumulated calcein in absence (white bar) or presence (black bar) of Pgp inhibitor PSC. Accumulation was significantly increased in ciPTEC-14.4 in presence of PSC (p<0.001). Data are expressed as mean values +/- SE of three experiments.

### Examples

### Example 1: Primary cell culture

Primary cells were cultured as described before by collecting mid stream urine after signing of informed consent by the parents of healthy volunteers with no clinical history of renal disease, nor with any other chronic disease. Urine sediment was transferred to supplemented DMEM-HAM's F12 medium (Lonza; Basel, Switzerland), and cultured at 37°C, 5% CO2 [30].

### Example 2: Immortalization and subcloning

Primary cells were infected with SV40T and hTERT vectors containing respectively geneticin (G418) or hygromycin resistance as described before [24,17]. Subconfluent cell layers were transferred to 33°C and selected using G418 (400 µg/ml; Sigma-Aldrich) and hygromycin B (25 µg/ml (Sigma-Aldrich)) for 10 days. To obtain a homogenous cell culture, cells were subcloned using irradiated NIH 3T3 fibroblast as non-dividing feeder cells [23]. After culturing for two weeks at 33°C, single cell clones were visible and picked using cloning discs drained in trypsin/EDTA. For the following experiments, cells were cultured at 33°C to 70% confluency, followed by maturation for 10 days at 37°C during which the cells formed a confluent monolayer. Propagation of cells was maintained by reseeding the cells at a dilution of 1:3 to 1:6 at 33°C. Experimental procedures were performed on the cloned cells between passages 15 and 40.

Morphology of ciPTEC-14.4 was investigated using phase contrast microscopy. Additionally, cells cultured for 10 days at 37°C were scraped off flask using a rubber policeman and embedded in paraffin for electron microscopy analysis.

### Example 3: Characterization of ciPTEC-14.4

To investigate the epithelial origin of cells, confluent monolayers were fixed using 2% paraformaldehyde, permeabilized in PBS-Tween (0.1 %) and incubated with antibodies against the tight junction protein ZO-1 (1:25 dilution; Zymed Laboratories, South San Francisco, CA, USA). Following secondary goat-anti-rabbit-Alexa488 conjugate (Dako, Glostrup, Denmark) and DAPI (Molecular Probes, Invitrogen) to stain nuclei, cells were analyzed using immuno-fluorescence microscopy. The presence of brush border membrane protein aminopeptidase N using mouse-antihuman CD13-FITC antibody (Dako) and endothelial marker CD31-FITC (Dako) was detected as described previously [30]. Additionally, a sample of stained cells was transferred to a glass slide by cyto-spin (1000 x g, 10 min) and analyzed using immuno-fluorescence microscopy. Alkaline phosphatase activity was determined in at least three independent experiments using BM Chemiluminescence ELISA substrate (AP) kit (Roche Diagnostics, Mannheim, Germany) as described before [30]. Values are compared to HK-2 cell line using shrimp alkaline phosphate as positive control and expressed as mean +/- SE.

To investigate whether the monolayers assembled sufficiently tight for transport studies, ciPTEC-14.4 was cultured on Transwell®-Clear polyester membranes (Corning Costar Corporation, Cambridge, MA, USA) for 10 days at 37°C. Both apical and basal compartments were washed in HEPES-Tris buffer (Hepes-Tris (10mM), NaCl (132mM), KCI (4.2mM), CaCl2 (1 mM), MgCl2 (1 mM), D-glucose (5.5mM), pH 7.4), prior to the addition of 0.1 mg/ml inulin-FITC (Sigma-Aldrich) to the apical compartment. Inulin-FITC diffusion through the monolayer was monitored for 2 hours by sampling 100µl of both apical and basal compartments and measuring fluorescence at 485 nm with emission at 535 nm. Data are expressed as mean +/- SE.

### Example 4: PAGE and Western Blotting

Cellular homogenates of cells cultured for various days at 37°C were made by scraping cells off using a rubber policeman from 75 cm² tissue culture flask and lysed in 400 µl RIPA buffer containing Igepal CA630 (1%), Na-deoxycholate (0.5%), sodium dodecyl sulfate (SDS) (0.1 %), phenylmethanesulphonylfluoride (PMSF) (0.01 %), aprotinin (3%) and Na-orthovanadate (1 mM). Expression of SV40T antigen in cell homogenates was analyzed by Western blotting using reduced 12% sodium dodecyl sulphate polyacrylamide gel electrophoreses (SDS-PAGE) and blotted onto a PVDF membrane (Immobilon, Millipore; Bedford, MA, USA). Membranes were incubated with SV40T antibody (1:400 dilution; Santa Cruz Biotechnology, Santa Cruz CA, USA) and GAPDH (1:5.000 dilution; Abcam, Cambridge, UK) as a house-keeping antigen, followed by incubation with goat-anti-mouse-HRP conjugate (Dako) and visualization using Pierce ECL Western blotting substrate (Thermo Fisher Scientific, Waltham MA, USA).

Cellular homogenates matured for 10 days at 37°C were analyzed as described above using 6 or 12% SDS-PAGE as indicated, using the following antibodies: rabbit anti-aquaporin 1 (AQP1; 1:4000; 12%; Chemicon Intl, Millipore), rabbit anti-OCT2 (1:500; 12%, Alpha Diagnostics, San Antonio TX, USA), rabbit anti-CD26 (dipeptidyl peptidase IV (dpp-IV); 1:200, 12%; Santa Cruz Biotechnology), rabbit anti-multidrug resistance protein 4 [27] (MRP4, ABCC4; 1:5000; 6%), mouse anti-Pgp (1:200; 6%; Dako) goat-anti-mouse-HRP conjugate (Dako) and goat-anti-rabbit-HRP conjugate (Dako). Human kidney homogenate in RIPA buffer was used as control.

### Example 5: Albumin uptake by endocytosis

The ability of ciPTEC-14.4 to reabsorb albumin was investigated by the incubation of confluent monolayers in 24 well plates with 50 µg/ml BSA-FITC (Sigma-Aldrich) for 30 min at 37°C unless described otherwise. Uptake was arrested using ice-cold PBS and cells were detached using trypsin, fixed by paraformaldehyde (0.5%) in PBS and analyzed using flow cytometry or immuno-fluorescence microscopy. Concentration- and temperature-dependent uptake, was investigated using a concentration range BSA-FITC (0; 3.7; 11; 33; 100; 300 µg/ml) at 37°C and on ice for 30 min. Uptake inhibition was studied in three independent experiments by incubating the cells with BSA-FITC (50 µg/ml) in addition of excess unlabelled BSA (10 mg/ml) or recombinant RAP (1 µM), which was a kind gift of Dr. M. Nielsen (University of Aarhus, Denmark). Uptake inhibition by RAP was further examined using a dilution range of RAP. BSA uptake in saturation experiments are plotted as mean fluorescence intensity and in inhibition experiments as mean (+/- SE) percentage uptake compared to control condition.

### Example 6: Sodium-dependent phosphate uptake

Phosphate uptake was performed in confluent monolayers using 32PO4 (Perkin Elmer, Waltham MA, USA) as described earlier [14]. Cells cultured for 10 days at 37°C were incubated with 0.2 mM KH2PO4 (10 µCi/ml) for 5 min in four independent experiments, in the presence of 137 mM sodium or 137 mM NMDG to study sodium-dependent transport. Additionally, time- (0.5; 1; 2; 5; 10; 15; 30 or 60 min) and concentration-dependent (0.02; 0.07; 0.22; 0.66 or 2 mM PO4) uptake was studied. Data are expressed as mean +/- SE.

### Example 7: OCT2/SLC22A2 activity

Transport of xenobiotics across the basolateral membrane was investigated in ciPTEC-14.4 by measuring the activity and expression of the solute carrier tansporter (SLC) OCT2/SLC22A2 using a method adapted from Brown et al [4]. Cells were grown on Transwell®-Clear polyester membranes as described before. Activity of OCT2 was measured by incubating 1 µM fluorescent OCT2 substrate 4-(4-(dimethylamino)styryl)-N-methylpyridinium iodide (ASP, Invitrogen) in Hepes-Tris buffer for 1 min at 37°C at the basal compartment. To inhibit OCT2 mediated uptake, cells were exposed to 100µM tetrapentylammonium (TPA) at both apical and basal compartments for 10min prior to uptake of ASP. Additionally, one set of experiments was performed at 4°C. After incubation, transport was arrested using 1 mM ice-cold TPA. Cells were homogenized using 250µl Hepes-Tris-Triton (0.1 %) buffer for 30 min, followed by analyzing fluorescence intensity (excitation 450 nm, emission 642 nm) using Victor3 Multiplate Reader (Perkin Elmer Inc.). Data are expressed as mean +/-SE.

### Example 8: Pgp/MDR1/ABCB1 activity

The activity of the ATP binding cassette (ABC) efflux transporter Pgp was assessed by measuring the accumulation of calcein as describe before [28]. Briefly, matured cells were incubated in two independent experiments for 1 hr at 37°C with lipophylic non-fluorescent Pgp substrate calcein-AM (Invitrogen) in the presence or absence of inhibitor PSC-833, which was a kind gift from Novartis Pharma (Basel, Switzerland). Intracellularly, calcein-AM is metabolized by esterase activity to the fluorescence calcein. Fluorescence of cell lysates was measured at 488 nm with emission at 518 nm. Fluorescence is expressed as mean +/- SE.

### Example 9: Statistical analysis

Michaelis-Menten curve fitting for calculation of Km and Vmax values was performed by non-linear regression analysis using GraphPad Prism 4.03 software. Differences in substrate transport in presence or absence of inhibitors or unlabelled analogues were assessed by a paired t-test.

### Example 10: Measuring the trans-epithelial electric resistance

The evaluation of the monolayer integrity was done by measuring the trans-epithelial electric resistance (TEER) expressed as ohms.cm². These were measured by using a Millicell-ERS TEERmeter at room temperature. The results are shown in Table 1.

**Table 1**

| | Control Membrane (1) | Type I rat collagen (1) | Fibronectin (1) | Laminin (1) | Type IV human Collagen (2) |
|---|---|---|---|---|---|
| Mean (Ohm cm²) | 288 | 284 | 271 | 280 | 212 |
| sd | 105 | 101 | 56 | 64 | 16 |

| | | | | | |
|---|---|---|---|---|---|
| (1) Polycarbonate supports with porosity of 0.45 micrometer. Passage No: 20 at 18 days post seeding. (2) Polycarbonate supports with porosity of 1 micrometer. Passage No: 35 at 18 days post seeding. | | | | | |

### References

1. Bens M, Vandewalle A (2008) Cell models for studying renal physiology. Pflugers Arch 457:1-15
2. Birn H, Christensen EI (2006) Renal albumin absorption in physiology and pathology. Kidney Int 69:440-449
3. Bodnar AG, Ouellette M, Frolkis M, Holt SE, Chiu CP, Morin GB, Harley CB, Shay JW, Lichtsteiner S, Wright WE (1998) Extension of life-span by introduction of telomerase into normal human cells. Science 279:349-352
4. Brown CD, Sayer R, Windass AS, Haslam IS, De Broe ME, D'Haese PC, Verhulst A (2008) Characterisation of human tubular cell monolayers as a model of proximal tubular xenobiotic handling. Toxicol Appl Pharmacol 233:428-438
5. Detrisac CJ, Mayfield RK, Colwell JA, Garvin AJ, Sens DA (1983) In vitro culture of cells exfoliated in the urine by patients with diabetes mellitus. J Clin Invest 71:170-173
6. Detrisac CJ, Sens MA, Garvin AJ, Spicer SS, Sens DA (1984) Tissue culture of human kidney epithelial cells of proximal tubule origin. Kidney Int 25:383-390
7. Dorrenhaus A, Muller JI, Golka K, Jedrusik P, Schulze H, Follmann W (2000) Cultures of exfoliated epithelial cells from different locations of the human urinary tract and the renal tubular system. Arch Toxicol 74:618-626
8. EI-Sheikh AA, van den Heuvel JJ, Krieger E, Russel FG, Koenderink JB (2008) Functional Role of Arginine 375 in Transmembrane Helix 6 of Multidrug Resistance Protein 4 (MRP4/ABCC4). Mol Pharmacol
9. Forster IC, Hernando N, Biber J, Murer H (2006) Proximal tubular handling of phosphate: A molecular perspective. Kidney Int 70:1548-1559
10. Gahl WA, Thoene JG, Schneider JA (2002) Cystinosis. New England Journal of Medicine 347:111-121
11. Gekle M (2005) Renal tubule albumin transport. Annu Rev Physiol 67:573-594
12. Gekle M, Mildenberger S, Freudinger R, Silbernagl S (1996) Functional characterization of albumin binding to the apical membrane of OK cells. Am J Physiol 271 :F286-F291
13. Madsen KM, Nielsen S, Tisher CC (2008) Proximal tubule. In: The Kidney, 8th ed., edited by Brenner BM, Philadelphia, WB Saunders, 2008, pp 39-50.
14. Malmstrom K, Stange G, Murer H (1988) Intracellular cascades in the parathyroid-hormone-dependent regulation of Na+/phosphate cotransport in OK cells. Biochem J 251:207-213
15. Nielsen S, Kwon TH, Christensen BM, Promeneur D, Frokiaer J, Marples D (1999) Physiology and pathophysiology of renal aquaporins. J Am Soc Nephrol 10:647-663
16. O'Brien J, Wilson I, Orton T, Pognan F (2000) Investigation of the Alamar Blue (resazurin) fluorescent dye for the assessment of mammalian cell cytotoxicity. Eur J Biochem 267:5421-5426
17. O'Hare MJ, Bond J, Clarke C, Takeuchi Y, Atherton AJ, Berry C, Moody J, Silver AR, Davies DC, Alsop AE, Neville AM, Jat PS (2001) Conditional immortalization of freshly isolated human mammary fibroblasts and endothelial cells. Proc Natl Acad Sci U S A 98:646-651
18. Racusen LC, Monteil C, Sgrignoli A, Lucskay M, Marouillat S, Rhim JG, Morin JP (1997) Cell lines with extended in vitro growth potential from human renal proximal tubule: characterization, response to inducers, and comparison with established cell lines. J Lab Clin Med 129:318-329
19. Racusen LC, Wilson PD, Hartz PA, Fivush BA, Burrow CR, Philip ET (1995) Renal Proximal Tubular Epithelium from Patients with Nephropathic Cystinosis - Immortalized Cell-Lines As In-Vitro Model Systems. Kidney International 48:536-543
20. Reshkin SJ, Forgo J, Murer H (1990) Functional asymmetry of phosphate transport and its regulation in opossum kidney cells: phosphate transport. Pflugers Arch 416:554-560
21. Russel FG, Masereeuw R, van Aubel RA (2002) Molecular aspects of renal anionic drug transport. Annu Rev Physiol 64:563-594
22. Ryan MJ, Johnson G, Kirk J, Fuerstenberg SM, Zager RA, Torok-Storb B (1994) HK-2: an immortalized proximal tubule epithelial cell line from normal adult human kidney. Kidney Int 45:48-57
23. Saleem MA, O'Hare MJ, Reiser J, Coward RJ, Inward CD, Farren T, Xing CY, Ni L, Mathieson PW, Mundel P (2002) A conditionally immortalized human podocyte cell line demonstrating nephrin and podocin expression. J Am Soc Nephrol 13:630-638
24. Satchell SC, Tasman CH, Singh A, Ni L, Geelen J, von Ruhland CJ, O'Hare MJ, Saleem MA, van den Heuvel LP, Mathieson PW (2006) Conditionally immortalized human glomerular endothelial cells expressing fenestrations in response to VEGF. Kidney Int 69:1633-1640
25. Stamps AC, Davies SC, Burman J, O'Hare MJ (1994) Analysis of proviral integration in human mammary epithelial cell lines immortalized by retroviral infection with a temperature-sensitive SV40 T-antigen construct. Int J Cancer 57:865-874
26. Terryn S, Jouret F, Vandenabeele F, Smolders I, Moreels M, Devuyst O, Steels P, Van KE (2007) A primary culture of mouse proximal tubular cells, established on collagen-coated membranes. Am J Physiol Renal Physiol 293:F476-F485
27. van Aubel RA, Smeets PH, Peters JG, Bindels RJ, Russel FG (2002) The MRP4/ABCC4 gene encodes a novel apical organic anion transporter in human kidney proximal tubules: putative efflux pump for urinary cAMP and cGMP. J Am Soc Nephrol 13:595-603
28. van de Water FM, Boleij JM, Peters JG, Russel FG, Masereeuw R (2007) Characterization of P-glycoprotein and multidrug resistance proteins in rat kidney and intestinal cell lines. Eur J Pharm Sci 30:36-44
29. Weiland C, Ahr HJ, Vohr HW, Ellinger-Ziegelbauer H (2007) Characterization of primary rat proximal tubular cells by gene expression analysis. Toxicol In Vitro 21:466-491
30. Wilmer MJG, Graaf-Hess A, Blom HJ, Dijkman HBPM, Monnens LA, van den Heuvel LP, Levtchenko EN (2005) Elevated oxidized glutathione in cystinotic proximal tubular epithelial cells. Biochemical and Biophysical Research Communications 337:610-614
31. Yang YS, Balcarcel RR (2004) 96-well plate assay for sublethal metabolic activity. Assay and Drug Development Technologies 2:353-361
32. Zhai XY, Nielsen R, Birn H, Drumm K, Mildenberger S, Freudinger R, Moestrup SK, Verroust PJ, Christensen EI, Gekle M (2000) Cubilin- and megalin-mediated uptake of albumin in cultured proximal tubule cells of opossum kidney. Kidney Int 58:1523-1533.

## Claims

1. A conditionally immortalized human proximal tubule cell line expressing MRP4/ABCC4 in concert with expressing active OCT2 and Pgp.

2. Cell line according to claim 1 with the following characteristics:
- Cobblestone morphology
- Expression of zona-occludens1 (ZO1), epitheliale tight junction marker
- Resistance of monolayer on permeable filters
- Transepithelial electrochemical resistance (TEER) of monolayer
- Expression of aminopeptidase N, enzyme on proximal brush border
- Alkaline phosphatase activity, enzyme on proximal brush border
- Expression of specific proximal tubular proteins:
○ aquaporin1 (AQP1)
○ organic cation transporter 2 (OCT2/SLC22A2)
○ dipeptidyl-peptidase IV (dppIV)
○ multidrug resistance protein 4 (MRP4/ABCC4)
○ P-glycoprotein (Pgp/MDR1/ABCB1)
- Albumine endocytosis, specifically inhibited by receptor associated protein (RAP)
- Sodium-dependent phosphate uptake
- Functional basolateral transport of substrate ASP via OCT2/SLC22A2
- Functional efflux transport of calceïne via P-glycoprotein/MDR1/ABCB1
- Proliferation of more than 40 passage numbers, while maintaining morphology and expression of AQP1, Pgp/MDR1/ABCB1 and OCT2/SLC22A2 and sodium-dependent phosphate uptake and Pgp-activity.

3. Cell line selected from the group consisting of cell lines as deposited with the DSMZ under accession numbers DSM ACC3019, DSM ACC3020, DSM ACC3021and DSM ACC3022

4. Use of a cell line according to claims 1 - 3 for the functional analysis of renal transporters.

5. Bioassay for testing transport properties of substances comprising a cell line according to claim 1.

6. Use of a cell line according to claims 1 - 3 in an (bio) artificial kidney for specific reabsorption of electrolytes from ultrafiltrate.

7. A permeable filter covered with a monolayer of cells obtained from a cell line according to claims 1 - 3.
